# EUROPEAN PATENT APPLICATION

(11) **EP 4 717 159 A1**
(43) Date of publication of application: **01.04.2026**
(21) Application number: 25204321.1
(22) Date of filing: 24.09.2025
(51) Int. Cl.: A61B 5/0531

(54) **EXAMINING VISIBLE SKIN CONDITIONS**

(30) Priority: 30.09.2024 GB 202414309
(71) Applicant: HM Technology International Limited, Harrow HA1 2EY (GB)
(72) Inventor: MAMIGONIONS, DR HRAND MAMI, LONDON, W5 3EB (GB)
(74) Representative: Atkinson & Company Intellectual Property Limited

(57) **Abstract**

A method for examining visible skin conditions is shown. A housing (101) is located at a region of a visible skin condition and image data of the skin over this region is captured. Electric fields (2001, 2002) are generated that penetrate the skin over the region, such that an application of the apparatus produces both image-related signals and electric field related signals for the same skin condition. Depth of skin penetration of the visible skin condition may be assessed by calculating the surface area of the visible skin condition from the image data and determining the volume of the visible skin condition from the electric field related signals and processing the calculated surface area in combination with the determined indication of volume to assess the depth of skin penetration. The electric field generating device may comprise a first dielectric substrate (301) having a hole (302) and the capturing step may be performed by a camera (501) viewing the visible skin condition through this hole.

## Description

The present invention relates to an apparatus of the type for examining visible skin conditions, comprising a housing locatable at a region of a skin condition, wherein said housing contains: a camera for capturing image data of the skin over said region; and an electric field generating device configured to generate electric fields that penetrate the skin over said region, such that an application of the apparatus produces both image related signals and electric field related signals for the same skin condition.

The present invention also relates to method of examining a visible skin condition, of the type comprising the steps of: locating a housing at a region of a visible skin condition; capturing image data of the skin over said region; and generating electric fields that penetrate the skin over said region, such that an application of the apparatus produces both image-related signals and electric field related signals for the same skin condition.

It is known to examine skin conditions visually, which may be achieved directly, possibly via a lens or indirectly by means of a camera. Suggestions have been made to the effect that a video camera included within a mobile phone could be deployed to achieve this. However, problems arise in terms of reliability and repeatability.

Proposals have been made for examining visible skin conditions using electric fields, However, problems arise if electrodes generating these electric fields are in contact with a skin condition itself.

According to a first aspect of the present invention there is provided an apparatus of the aforesaid type, characterised in that: said electric field generating device comprises a first dielectric substrate with a hole having a first radius; and said camera views the skin condition through said hole.

In an embodiment, a processor configured to: assess the depth of skin penetration of the visible skin condition; and generate an output indicative of said assessing step. The processor may be configured to: calculate the surface area of the visible skin condition from the image data; determine an indication of the volume of the visible skin condition from the electric field related signals; and process said calculated surface area in combination with said determined indication of volume to assess the depth of skin penetration.

According to a second aspect of the present invention there is provided a method of the aforesaid type, characterised by assessing depth of skin penetration of the visible skin condition; and generating an output indicative of said assessing step, by the steps of: calculating the surface area of the visible skin condition from the image data produced by said capturing step; determining an indication of the volume of the visible skin condition from the electric field related signals; and processing said calculated surface area in combination with said determined indication of volume to assess the depth of skin penetration.

In an embodiment said generating step is performed by an electric field generating device that comprises a first dielectric substrate having a hole with a first radius; said capturing step is performed by a camera; and said camera views the visible skin condition through said hole.

Embodiments of the invention will be described, by way of example only, with reference to the accompanying drawings. The detailed embodiments show the best mode known to the inventor and provide support for the invention as claimed. However, they are only exemplary and should not be used to interpret or limit the scope of the claims. Their purpose is to provide a teaching to those skilled in the art. Components and processes distinguished by ordinal phrases such as "first" and "second" do not necessarily define an order or ranking of any sort. In the drawings:
*Figure 1* shows an apparatus for examining visible skin conditions;
*Figure 2* shows the apparatus of *Figure 1* obtaining test data;
*Figure 3* shows the apparatus of *Figure 1* in an inverted position;
*Figure 4* shows the apparatus of *Figure 1* in a cradle;
*Figure 5* details the dielectric substrate identified in *Figure 3*;
*Figure 6* shows an internal subassembly for the apparatus identified in *Figure 1**;*
*Figure 7* shows procedures performed by a microcontroller contained within the apparatus;
*Figure 8* details procedures for producing electric field data as identified in *Figure 7*;
*Figure 9* shows procedures for calculating and saving data identified in *Figure 8**;*
*Figure 10* illustrates the production of output samples identified in *Figure 9**;*
*Figure 11* details procedures for identifying and storing information identified in *Figure 9*;
*Figure 12* illustrates the population of a database table;
*Figure 13* shows the examining apparatus connected to a data processing system;
*Figure 14* shows procedures performed by the data processing system identified in *Figure 13*;
*Figure 15* details procedures for processing the received data identified in *Figure 14*;
*Figure 16* illustrates the processing of images;
*Figure 17* shows an image of a first visible skin condition;
*Figure 18* shows an image of a second visible skin condition
*Figure 19* shows a procedure for processing image and electric field related data; and
*Figure 20* shows a cross section of the apparatus of Figure 3.

### Figure 1

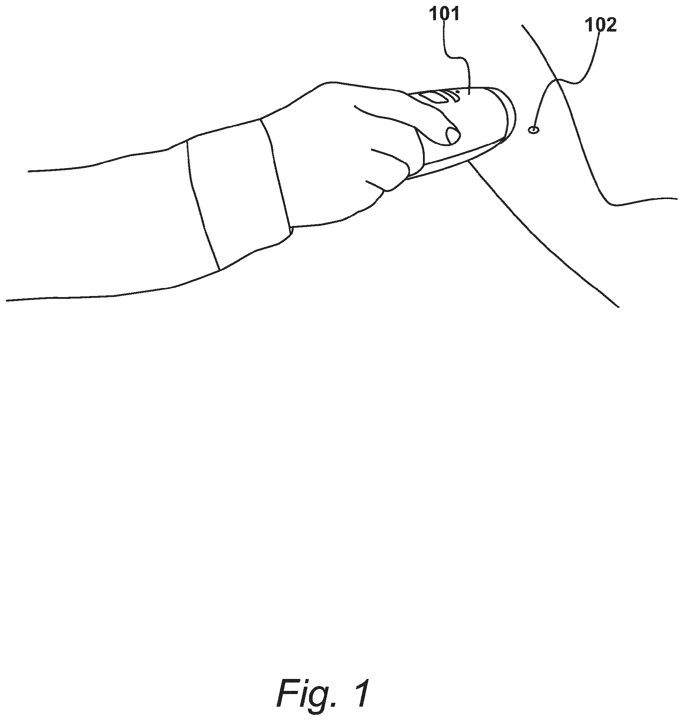

An apparatus for examining visible skin conditions is shown in *Figure 1* and comprises a housing **101** locatable at a region of the skin under examination; an example **102** of a skin condition is illustrated. The housing contains a camera for capturing image data of the skin over the region of the skin condition. In addition, an electric field generating device is configured to generate electric fields that penetrate the skin over the region, such that an application of the apparatus produces both image related signals and electric field related signals for the same skin condition.

In an embodiment, reference image data is produced and reference electric field data is also produced by placing the apparatus firstly at a position of healthy skin, as shown in *Figure 1*. Thus, while located as illustrated in *Figure 1*, a camera captures reference image data and the electric field generating device produces reference signals from which electric field reference data is produced.

In an alternative embodiment, reference data is obtained by deploying the apparatus upon a reference proxy device having known optical and electrical properties. As an example, the reference proxy device may comprise a receptacle containing an oil, such as olive oil.

### Figure 2

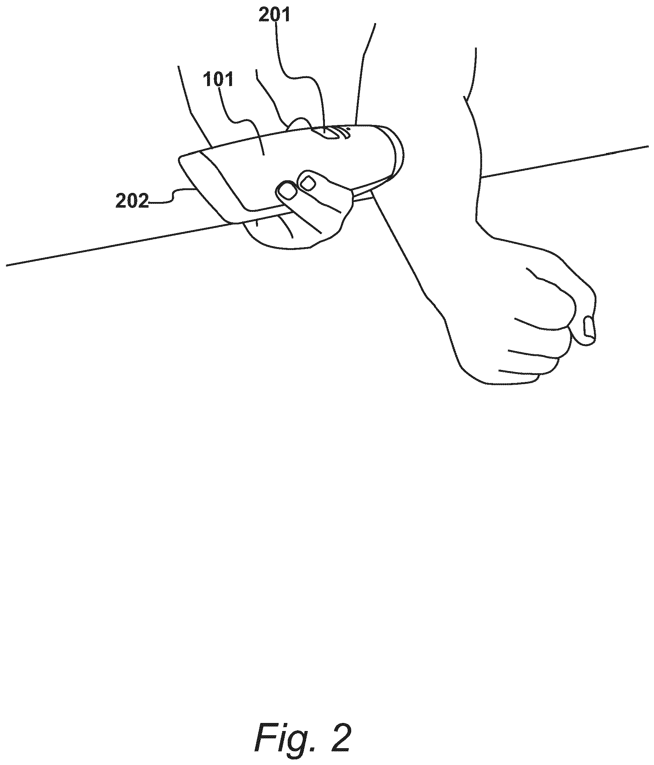

As shown in *Figure 2**,* after obtaining reference data, as described with respect to *Figure 1*, the apparatus is moved to the position of the visible skin condition **102** to produce test image data and test electric field data. To initiate a procedure, an operative applies pressure to a manual activation button **201** to generate reference data. Thereafter, after moving the apparatus to a location substantially similar to that shown in *Figure 2**,* a second activation of this button **201** results in the generation of the test data. In alternative embodiments, alternative measures may be taken to initiate this activation and activation could be initiated by a connected computer.

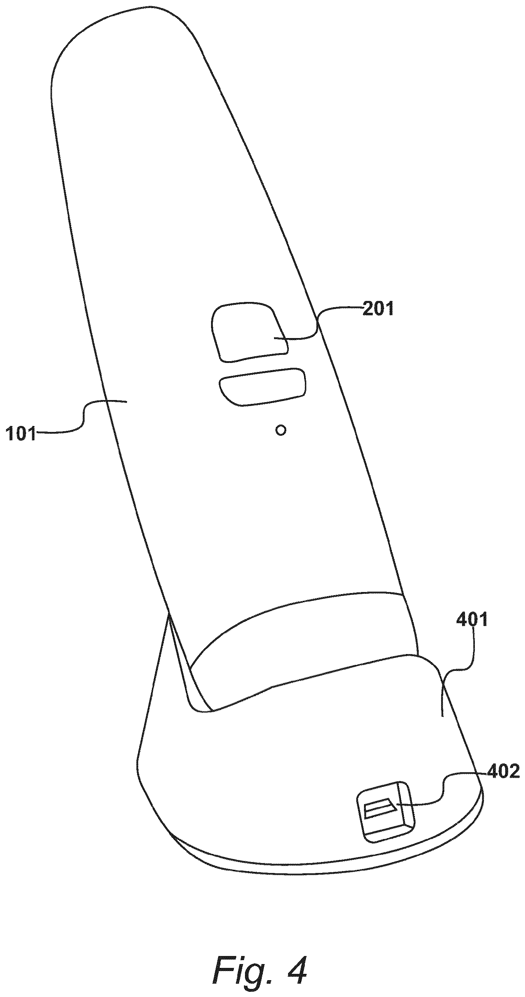

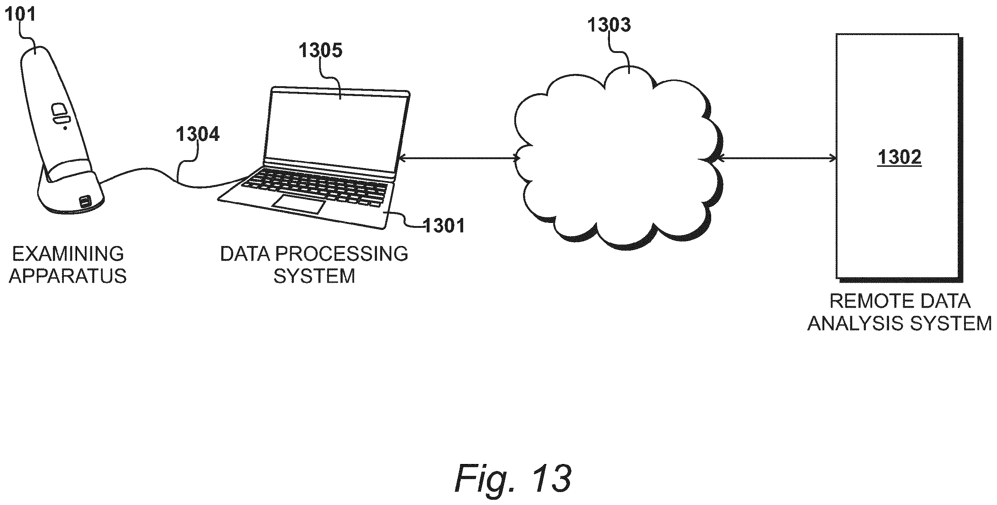

In the embodiment shown in *Figure 2**,* the apparatus includes a socket **202** for interfacing with a cradle, as described with reference to *Figure 4**,* which may in turn be connected to a data processing system, such as a laptop computer and a connection of this type will be described with reference to *Figure 13**.*

In the embodiment of *Figure 2**,* the camera is configured to capture image data substantially at the same time as the electric field generating device generates electric fields. In practice, these operations are likely to be performed sequentially but a complete cycle may be completed within a time interval of only a few seconds. Thus, from the perspective of an operative, the electric field data and the image data are captured substantially at the same time, such that the captured data is derived from the apparatus held in a fixed location relative to the region of the skin being examined or the region of the skin providing reference data.

### Figure 3

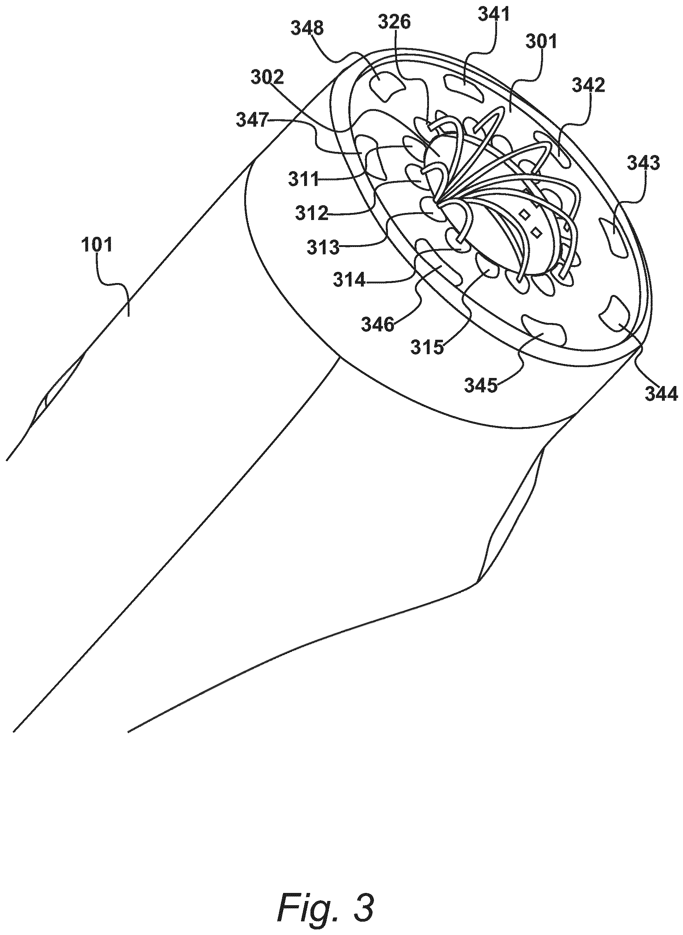

The housing **101** is shown inverted in *Figure 3**,* exposing a first dielectric substrate **301** with a hole **302** having a first radius. The dielectric substrate may take the form of a board of a substantially conventional type onto which circuits may be printed or etched. Scanning electrodes, including a first scanning electrode **311** and a second scanning electrode **312,** are circumferentially evenly displaced on a first planar surface of the dielectric substrate **301** at a second radius around the hole **302.** In this way, it is possible for the camera to view the skin condition through the hole **302.** This portion of the housing, which comes into contact with a subject's skin, may be covered by a transparent replaceable cap that is also configured to permit the transmission of electric fields.

In the embodiment shown in *Figure 3**,* the apparatus has a total of sixteen scanning electrodes, although alternative embodiments could have fewer or more scanning electrodes. In an embodiment, each scanning electrode **311** to **316** may be energised as a transmitter or monitored as a receiver. Thus, in an embodiment, a full scanning cycle may consist of energising each of the available scanning electrodes which are sequentially selected. In an embodiment, upon selecting a scanning electrode as a transmitter, it may be energised a total of fifteen times with a different scanning electrode being selected as the receiver for each energisation.

In the embodiment of Figure 3, each scanning electrode is configured exclusively to be energised, and thus act as a transmitter, or to be configured as a receiver. In the embodiment of Figure 3, the functionality of the electrodes is fixed; with eight of the scanning electrodes acting as transmitters alternating with the remaining eight electrodes acting as receivers. In the example shown in *Figure 3**,* electrode **331** is being energised sequentially eight times. Electrode **312** acts as a receiver and electrode **332** acts as a receiver on the next energisation cycle. Thus, eight energisations of the same electrode are made sequentially with each of the available eight receiving electrodes. Energisations of this type produce electric fields that extend away from the plane of the dielectric substrate **301.**

In the embodiment of *Figure 3**,* respective electrical conductors for each of the scanning electrodes **311 to 326** are configured to pass through the first dielectric substrate to a second planer surface.

The embodiment of *Figure* 3 also includes circumferentially and substantially evenly displaced secondary electrodes **341, 342** etc. In the embodiment of *Figure 3**,* a total of eight secondary electrodes (**341** - **348**) are provided at a third radius that is larger than the second radius. In an alternative embodiment, the number of secondary electrodes present is the same as the number of scanning electrodes and each secondary electrode may be radially displaced from a respective scanning electrode. The secondary electrodes **341** to **348** do not perform scanning operations as such and the scanning operations are performed exclusively by the scanning electrodes. However, appropriate connections to the secondary electrodes can influence the resulting electric fields and as such can increase the size of the dataset.

### Figure 4

After performing the operations described with respect to *Figure* 1 and *Figure 2**,* the apparatus is returned to a cradle **401.** In an embodiment, the socket **202** engages a plug extending from the cradle **401** and the cradle **401** may be connected to a data processing system by means of a USB (or similar) socket **402.** A USB connection from socket **402** may be used to perform data transfer from the apparatus to a data processing system.

In an alternative embodiment, data transfer from the apparatus to the data processing system occurs wirelessly, thereby enhancing an operative's ability to move around a subject and position the apparatus appropriately.

### Figure 5

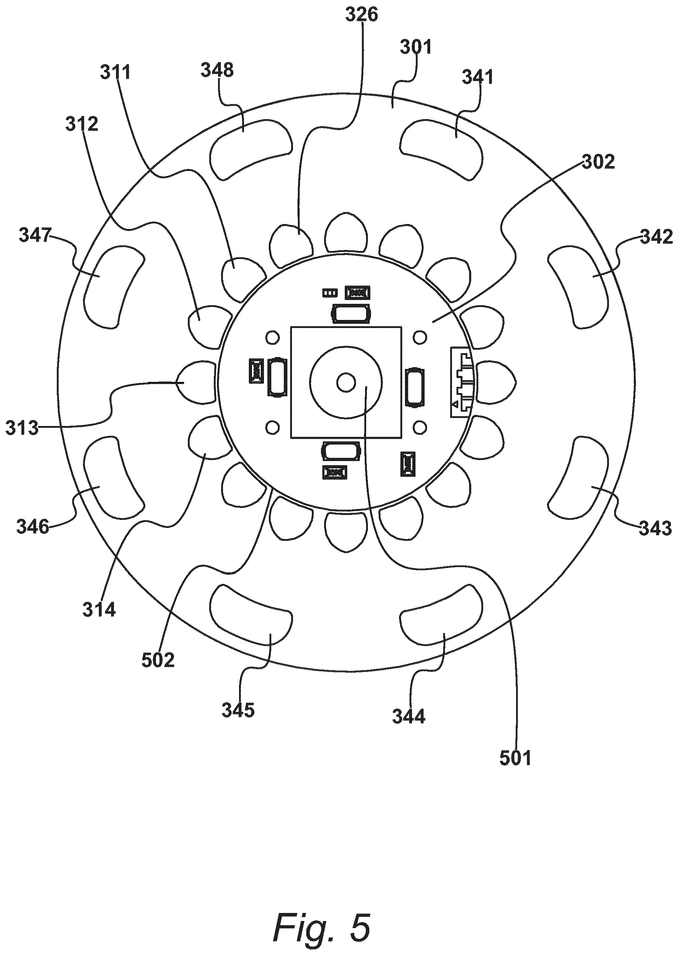

The dielectric substrate **301** is shown in *Figure 5*, with the scanning electrodes **311** to **316** and the secondary electrodes **341** to **348** mounted thereon. A camera **501** is visible through the hole **302** and is surrounded by a cylindrical shroud **502.**

The camera **501** may produce individual still images in response to being triggered. Alternatively, the camera may be a video camera producing a stream of video images which may be viewed on an appropriate external device while the apparatus is being placed in position. Activation of the manual activation button **201** then results in a particular image being selected followed by the electric fields being generated to produce electric field data.

In an alternative embodiment, it is also possible for video material to be recorded.

### Figure 6

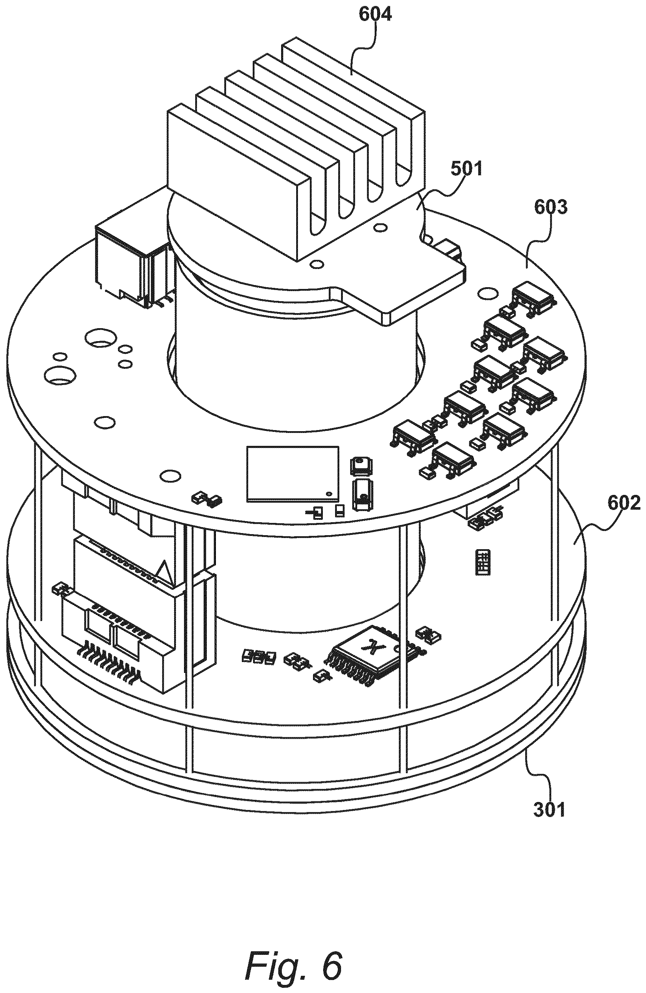

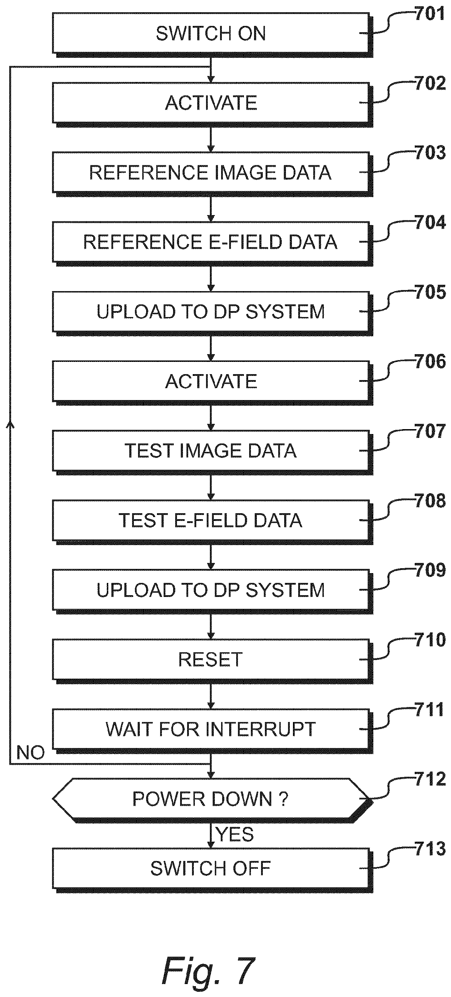

An internal subassembly for the apparatus is shown in *Figure 6*, with the first dielectric substrate **301** inverted. The first dielectric substrate **301** may take the form of a conventional circuit board which, as shown in *Figure 6**,* is attached to a second circuit board **602** which is in turn attached to a third circuit board **603.** Electronic components are supported on the second circuit board **602** and a third circuit board **603** for implementing procedures described with reference to *Figure* 7 to *Figure 12*. In an embodiment, these electronic components include an inertial module such as that produced by iNEMO and made available under the commercial designation LSM6 DS016IS. This provides a three-axis accelerometer and a three-axis gyroscope with an intelligent sensor processing unit. In this way, it is possible for a data processing system to receive data indicating the position and orientation of the apparatus when in use as described with reference to *Figure 1* and *Figure 2**.*

The underside of camera **501** is also shown in *Figure 6*, to which is attached a heatsink **604**. In an embodiment, the camera is widely available under the commercial designation OV5640 and is capable of providing image data at a definition from 320×240 pixels up to 2592x1944 pixels in an eight-bit or ten-bit RAW RGB output format. An autofocusing capability is also available but the necessity for this is mitigated on the basis that the distance between the camera and the subject remains substantially the same; given that activation only takes place after the apparatus has been appropriately located.

### Figure 7

Procedures performed by a microcontroller contained within the apparatus described with reference to *Figure 6**,* are detailed in *Figure 7**.* After being switched on at step **701,** the apparatus is placed in a reference position, as described with reference to *Figure 1*. The processor is then interrupted by activation of the manual activation button **201** at step **702.** In an alternative embodiment, it is possible for a first thread to be responsible for generating image data and a second thread to be responsible for generating the electric field data. In the embodiment shown in *Figure 7**,* the reference image data is produced at step **703** and the reference electric field data is then produced at step **704.** In an embodiment, a light-emitting diode present within the apparatus may be activated to indicate to an operative that the reference data has been collected. The operative would then be invited to relocate the apparatus to collect the test data, as described with reference to *Figure 2**.*

Meanwhile, in an embodiment, the reference image data and the reference electric field data are uploaded to a data processing system as described with reference to *Figure 13*. Thus, the light-emitting diode may be illuminated after the upload process has been completed and the procedure then anticipates a further activation of the manual activation button **201** at step **706.**

At step **707,** test image data is produced which is then followed, at step **708,** by the production of test electric field data. These procedures are substantially similar to procedures **703** and **704** respectively. Thus, again, the test image data and the test electric field data are uploaded to the data processing system at step **709.**

In the embodiment of *Figure* 7, a reset operation is performed at step **710** in anticipation of a further interrupt signal being received. The processor therefore enters a wait state at step **711.**

At step **712,** a question is asked as to whether the system is to power down and if answered in the affirmative, the apparatus switches off at step **713** to conserve battery power. Alternatively, if the question asked at step **712** is answered in the negative, the processor awaits the next activation interrupt at step **702.**

### Figure 8

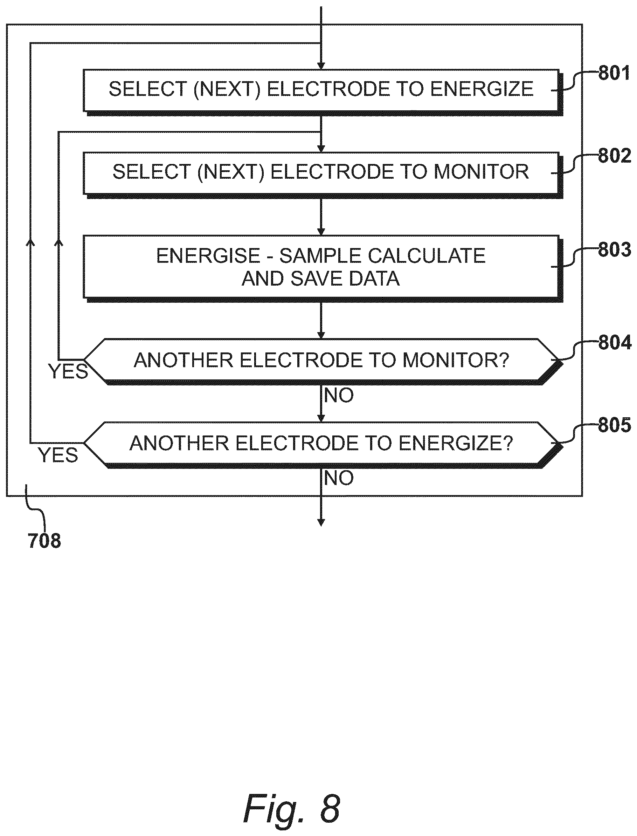

As described with reference to *Figure* 7, procedure **704** for producing the electric field reference data and procedure **708** for producing the electric field test data are substantially similar; and procedure **708** is detailed in *Figure* 8. In an embodiment, scanning electrodes may be selected for energisation to optimise the quality of the collected data. Similarly, appropriate scanning electrodes may be monitored. In the embodiment illustrated in *Figure 8**,* all of the transmitter electrodes are selected as an energised electrode and for each selected energised electrode, all of the remaining receiver electrodes are sequentially selected as monitored electrodes. The selection of these capacitively coupled electrodes may occur in any order but to facilitate the creation of appropriate instructions, the electrodes are selected sequentially in numerical order in the embodiment shown in *Figure 8**.*

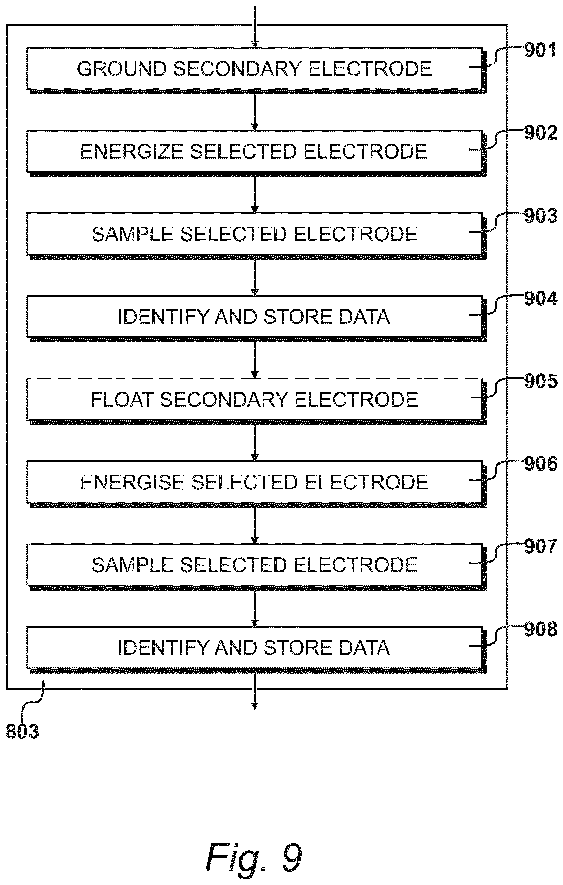

In the embodiment shown in *Figure 8**,* on a first iteration, the first scanning electrode **311** is selected at step **801** and the second scanning electrode **312** is selected at step **802.** At step **803,** data is collected for this capacitively coupled pair as described with reference to *Figure 9**.*

At step **804,** a question is asked as to whether another electrode is present to be monitored and on the first iteration this question will be answered in the affirmative resulting in the selection of the next receiver electrode. Thus, this procedure is repeated until all of the remaining receiver electrodes have been selected, resulting in the question asked at step **804** being answered in the negative.

At step **805,** a question is asked as to whether another transmitter electrode is present to be energised such that, on this first iteration, the next transmitter electrode will be selected at step **801** and repeated iterations of step **802** will result in all of the remaining receiver electrodes being sequentially selected as electrodes to be monitored. Thus, the process will continue until all of the transmitter electrodes have been selected at step **801** and the question asked at step **805** will be answered in the negative.

### Figure 9

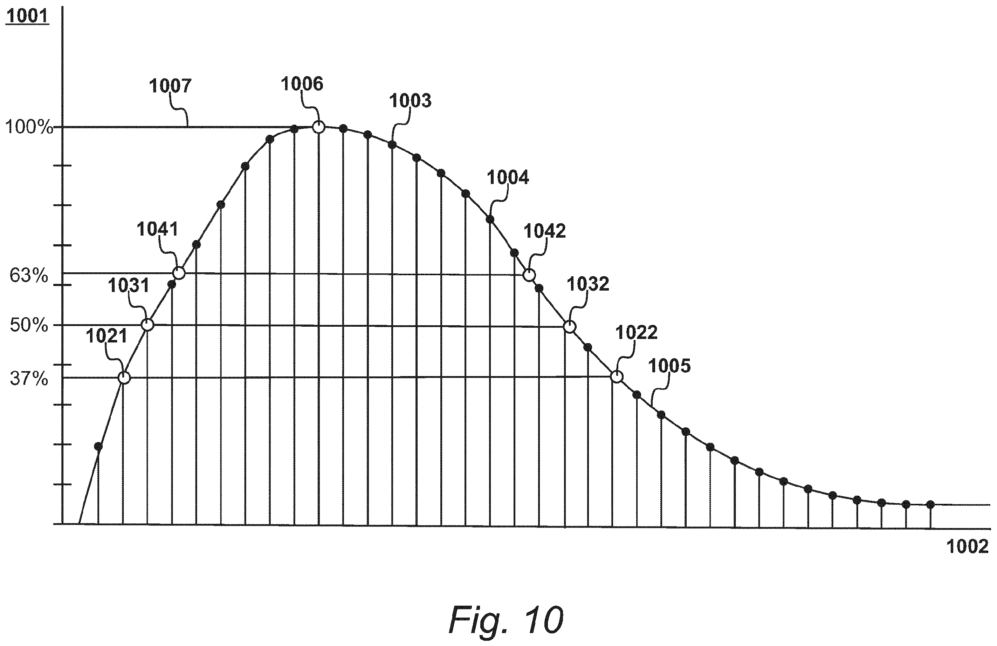

Procedure **803** for calculating and saving data derived from the electric field signals are detailed in *Figure 9*. At step **901,** one or more of the secondary electrodes **341** to **348** are grounded. At step **902** the scanning electrode selected at step **801** is energised and at step **903** the scanning electrode selected at step **802** is monitored and sampled. The data collected, as described with reference to *Figure 10**,* is then identified and stored at step **904.**

At step **905,** the secondary electrodes **341** to **348** are allowed to float and the selected electrodes are again coupled by energising the electrode selected at step **801** and monitoring the electrode selected at step **802.** Thus, each selected pair are capacitively coupled twice in accordance with this embodiment. Again, at step **908,** the data is identified and stored.

In an embodiment, each energising pulse lasts for a duration of ten microseconds and individual pulses are separated by a duration of ninety microseconds. During each cycle, one hundred samples are taken, thereby requiring a sample rate of five megahertz. In an alternative embodiment, fewer samples are taken and in alternative embodiments more samples may be taken. In an embodiment, fifty samples are taken.

An analogue to digital converter, which in an embodiment forms part of the microcontroller, converts each sample into a twelve-bit representation and as a result of this, each energising pulse generates a significant amount of data. However, the processor is fast enough to allow a significant amount of processing to take place during the sample period. Thus, by comparing samples, it is possible to identify a peak value and the regular intervals between samples allows the time at which this peak value occurred to be determined. Thus, each sample point is made up of data that defines a voltage level at a particular time.

The resulting data is saved initially at step **904** and then again at step **908.** This raw data is processed to produce a smaller volume of output data that, in an embodiment, is transferred to a data processing system as described with reference to *Figure 13**.*

In an alternative embodiment, the raw data is transmitted to the data processing system and the procedures identified above, for reducing the data volume, are performed on the data processing system.

### Figure 10

The production of output samples at step **903** (or at step **907**) is illustrated in *Figure 10**.* In the graph shown in *Figure 10**,* output voltage **1001** is plotted against time **1002.** The sampling operation creates data points, such as data point **1003** and data point **1004.** As is known in the art, it is also possible to fit a curve **1005** to the real data points, such that other values on this curve may be calculated through a process of interpolation. From this, it is possible to identify a peak value **1006.**

Having calculated the peak value **1006,** it is then possible to identify points at which a portion of this peak value has been achieved. Thus, from the large volume of raw data, it is possible to calculate a much more limited volume of data (information) which conveys what is required in terms of a rate of charge, a peak and a rate of discharge. Furthermore, it is known that the absolute peak value and the rate of discharge are directly related to the conductivity and permittivity of the tissue being examined.

### Figure 11

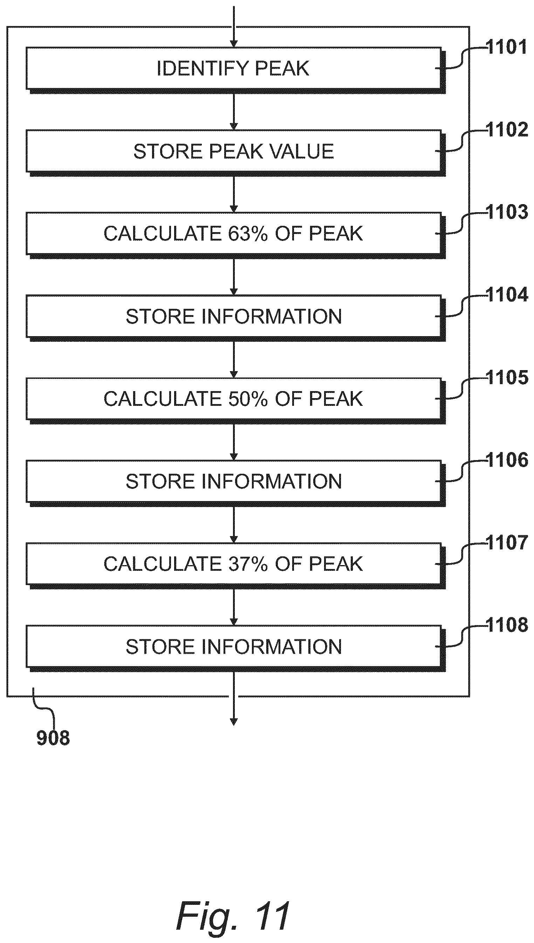

Procedures **904** and **908** for identifying and storing information (the data that will be uploaded to the data processing system) are shown in *Figure 11**.*

At step **1101,** the peak value **1006** is identified and this peak information **1007** is stored at step **1102.** At step **1103,** a value is calculated that represents sixty-three percent of the peak value **1007.** At step **1104,** data is stored, consisting of a first data point **1021** and a second data point **1022** at which the curve **1005** passes through the sixty-three percent value. At step **1105,** a value is calculated that represents fifty percent of the peak value **1007.** A first data point **1031** and a second data point **1032** are identified where the curve **1005** crosses this fifty percent level. At step **1107,** a value is calculated that represents thirty-seven percent of the peak value **1007.** Again, at step **1108,** a first data point **1041** is stored, along with a second data point **1042,** showing where the curve **1005** crosses these values.

### Figure 12

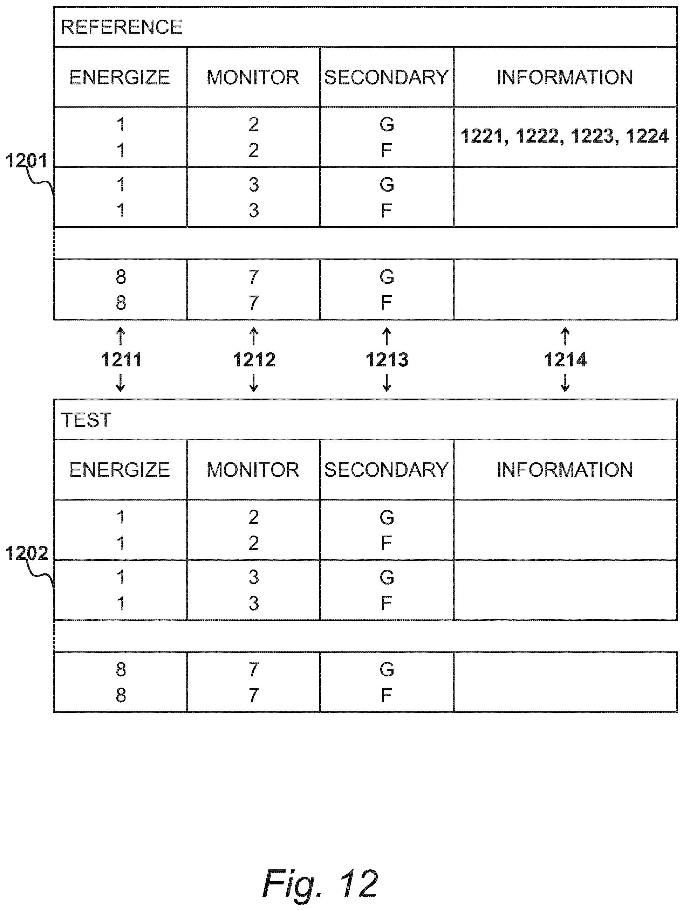

The refined data (or information) calculated at steps **1102, 1104, 1106** and **1108** are stored in a database and a representation of this database is illustrated in *Figure 12*. A data table **1201** is constructed for the reference data and a similar data table **1202** is constructed for the test data. In a first column **1211,** the electrode being energised is recorded and in a second column **1212** the electrode being monitored is recorded. For each of these combinations, a third column **1213** records whether secondary electrodes were grounded or allowed to float. The resulting information is then stored in a fourth column **1214.**

In this embodiment, the information (data for transmission to the data processing system) represents the first peak value **1221.** The information then represents the two positions for thirty-seven percent of the peak value **1222,** the two positions for fifty percent of the peak value **1223** and the two positions for sixty-three percent of the peak value **1224.**

After fully populating the database table of *Figure 12*, the resulting information consists of a relatively small volume compared to the totality of raw data generated through the scanning and sampling operations. In an embodiment, this information is transferred to a data processing system, as described with reference to *Figure 13*, for subsequent processing. The overall objective is to identify the nature of the tissue under consideration. In particular, when scanning visible skin conditions, the overall objective is to give an indication as to whether the skin condition is considered benign or whether the skin condition is considered malignant and therefore requires further attention.

As is clear from the table shown in *Figure 12*, similar combinations exist for both the reference stage **704** and the test stage **708.** Thus, specific similar information entries may be directly compared to determine the extent to which they differ. In an embodiment, a larger difference may indicate that the skin under test has characteristics that differ significantly from healthy skin; thereby prompting further investigation.

### Figure 13

The examining apparatus **101** is shown in *Figure 13*, connected to a data processing system **1301.** The data processing system **1301** also communicates with a remote data analysis system **1302** via the Internet **1303.**

In the embodiment described with reference to *Figure 6*, the apparatus includes a signal processor for processing electric field signals produced by the electric field generating device, which are in turn influenced by the electrical properties of the penetrated tissue, to produce electric field data as described with reference to *Figure* 7 to *Figure 12*. Thus, in an embodiment, the data stored in the database described with reference to *Figure 12* is transmitted to the data processing system ***1301*** either wirelessly or via a connected cable **1304.** The cable **1304** may also be used to recharge batteries contained within the apparatus **101.**

In an embodiment, data communication occurs wirelessly from the examining apparatus to the data processing system **1305** without the need for the examining apparatus to be returned to a cradle. In this way, it is possible for the examining apparatus **101** to be displaced by a significant distance away from the data processing system; possibly being located in a different room, while allowing the data to be collected by the data processing system. In this alternative embodiment, the examining apparatus is returned to a cradle for charging purposes only and cable **1304** may be connected to a conventional power supply.

In addition to transferring the electric field data, image data is also transmitted from the apparatus **101** to the data processing system **1301.** The data processing system **1301** provides a visual display **1305** for displaying the received image data.

The camera **501** may be a video camera and may continually supply video images to the data processing system, which can be viewed on the visual display **1305.** After positioning the apparatus, as described with reference to *Figure* 1 and *Figure 2**,* and upon activation of the manual activation button **201,** a high definition still image may be viewed on the visual display **1305.** In addition, a display may also be provided to show a graphical representation of the received electric field data. Furthermore, in an embodiment, it is possible for a visual image derived from the image data to be enhanced in response to the electric field data. This enhancement may be used to provide a local indication of the extent to which the skin condition may be considered malignant and therefore in need of further investigation. The electric field data and the image data may also be conveyed to the remote data analysis system **1302.**

In an embodiment, a machine learning exercise may be performed to analyse image data and electric field data in combination with independent assessments; such that it is possible to provide an additional indication of a need for medical intervention. Furthermore, over a period of time, as more data is collected, enhancements may be made to the machine learning process and the output from the machine learning process may be combined with local assessments made by operatives. This combined data may then allow enhancements to be made to the procedures performed by the data processing system **1305** and the remote data analysis system **1302.**

In an alternative embodiment, data collected by the examining apparatus **101** may be conveyed, possibly via a data processing system, directly to the remote data analysis system. This allows sophisticated analysis to take place, possibly derived from a machine learning process, and in turn an indication may be returned from the remote data analysis system to the examining apparatus to indicate whether the skin condition under consideration may be considered as benign or malignant.

In in an embodiment, appropriately coloured light emitting diodes may be included on the examination apparatus, resulting in the examining apparatus, for example, being illuminated in a blue colour if the area under consideration is considered to be benign and illuminated in a red colour if the area under consideration is considered to be malignant and thereby requiring further attention. Further colours may be included to indicate gradations between these two extremes. Alternatively, it is possible for a display device to be included as part of the examining apparatus itself.

### Figure 14

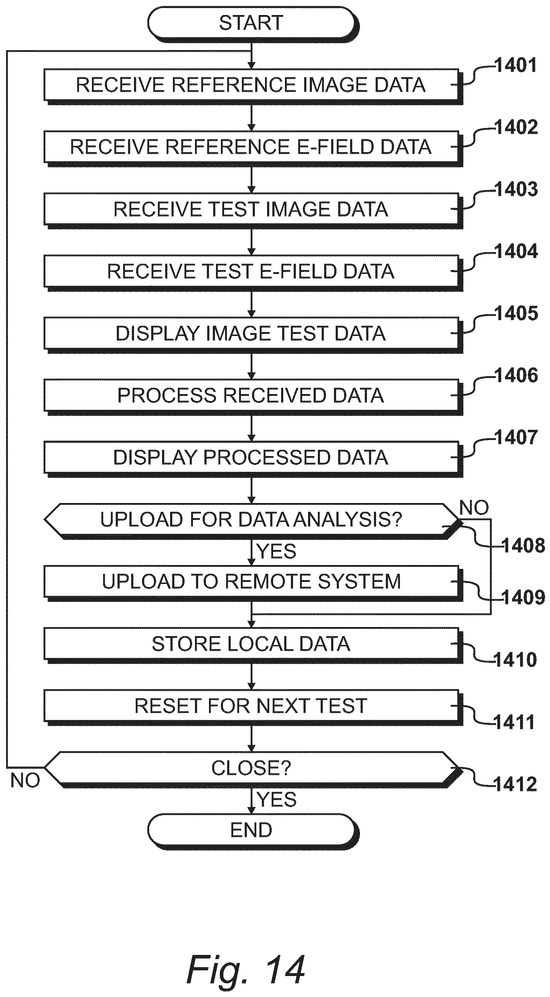

Procedures performed by the data processing system **1301** are illustrated in *Figure 14*. At step **1401,** reference image data is received whereafter, at step **1402,** reference electric field data is received. The selected images are stored in the examination apparatus **101** and the electric field data is read from the database table described with reference to *Figure 12**.* Thereafter, at step **1403,** test image data is received and at step **1404** test electric field data is received.

At step **1405** the image test data is displayed on the visual display **1305.** The test image data is captured in response to operation of the manual activation button **201** as described with reference to *Figure 2**.*

At step **1406** the data that has been received at steps **1401 to 1404** are processed and the processed data is displayed at step **1407.**

At step **1408** a question is asked as to whether the data is to be uploaded for data analysis and when answered in the affirmative, the data is uploaded to the remote data analysis system **1302.** This allows an operative to perform the exercise several times upon the same subject until data has been collected that is considered optimum by the operative. Thus, a graphical user interface displayed on the visual display **1305** may invite an operative to upload the data for data analysis or invite the operative to repeat the examination process.

At step **1410** the local data that has been uploaded to the remote system, at step **1409,** is stored locally on the data processing system **1301** whereafter, at step **1411,** the system is reset for the next testing operation to be performed. At step **1412** a question is asked as to whether the process is to close and the process ends if the question is answered in the affirmative. Alternatively, control returns to step **1401** in anticipation of receiving further data.

### Figure 15

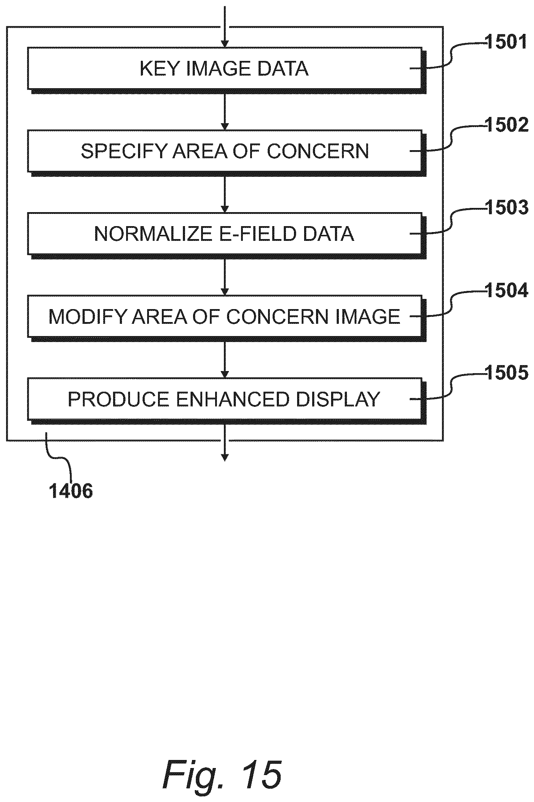

An example of procedure **1406** for processing the received data is shown in *Figure 15**.* However, it should be appreciated that many manipulations of this data may be performed to facilitate local assessments and to optimise the quality of the data that is uploaded to the remote data analysis system.

In this example, the image data is keyed at step **1501** to separate the image of the visible skin condition from background skin. This allows the area of concern to be specified at step **1502.**

At step **1503** the electric field data is normalised, which may involve subtracting the reference data from the test data. Again, alternative mathematical manipulations may be performed at this stage with a view to optimising the effectiveness of the electric field data.

Fundamentally, the purpose of these procedures is to identify the existence of problematic conditions. Thus, the electric field data may suggest that the area of skin which shows a visible skin condition does not represent an area of skin that may be considered problematic, which may in turn suggest that no further action is required. Alternatively, the electric field test data may be substantially different from the electric field reference data and this in turn may indicate that the skin condition is malignant and requires further attention. Consequently, in this embodiment, the area of concern in the image data is modified at step **1504** and an enhanced display of this modified data is produced at step **1505.**

### Figure 16

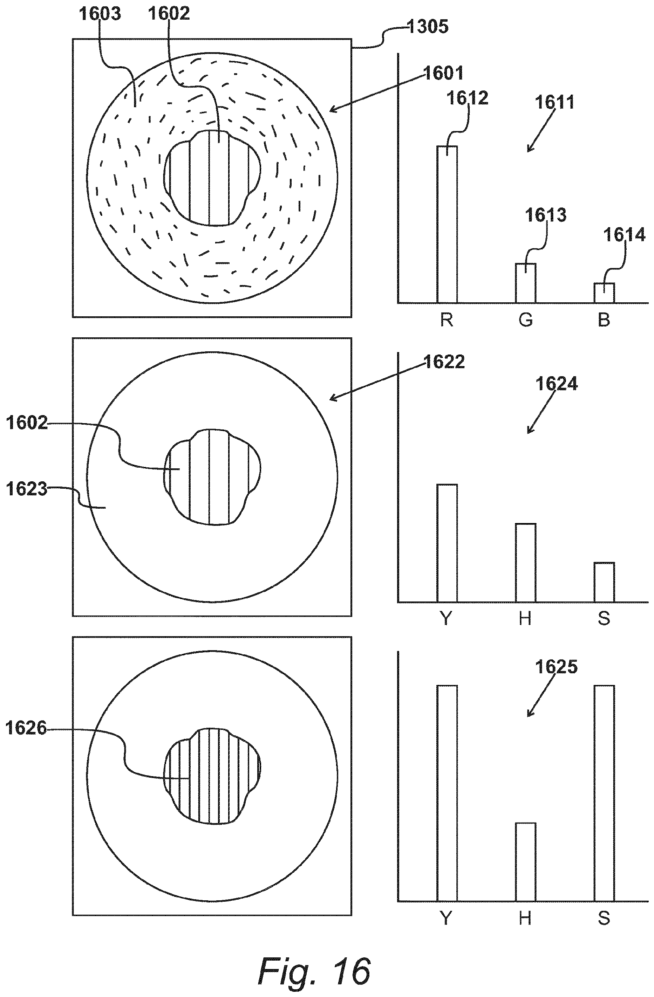

The results of the procedure described with reference to *Figure 15* are illustrated in *Figure 16*. Images are displayed on the visual display **1305,** either sequentially, as shown in *Figure 16*, or in combination.

A first image **1601** represents the image test data displayed at step **1405.** This shows the particular skin condition of interest **1602** surrounded by healthy skin **1603.** The raw image data consists of eight or ten bits representing light intensities for red, green and blue. These are illustrated in a first histogram **1611,** depicting a level for red **1612,** a level for green **1613** and a level for blue **1614.** These levels represent the colour of the skin condition **1602** and different levels would be represented for the surrounding skin **1603.** These differences allow the area representing the skin condition **1602** to be distinguished over values representing the background skin **1603.** Following known keying techniques, this allows pixels representing the skin condition to be separated from the background pixels, such that the image may depict the skin condition against a plain white background as shown in a second image **1622.** In the second image **1622,** the skin condition image **1602** appears as before but this time it is shown against a white background **1623.**

When represented in red-green-blue (RGB) colour space, pixel values representing the skin condition **1602** remain the same as illustrated in chart **1611.** As is known in the art, manipulations may be performed within this RGB colour space.

In this embodiment, the intention is to enhance pixel values which show the skin condition to an extent determined by the analysis of the electric field data. In this example, individual pixel values are transformed from red-green-blue colour space to luminance, hue and saturation colour space (usually identified as YHS respectively). A second histogram **1624** shows the same colour as that represented in chart **1611** but in YHS colour space. Furthermore, in this embodiment, modifications are made at step **1504** by adjusting the luminance values and the saturation values in response to the electric field data.

For the purposes of this example, it is assumed that the electric field test data differs significantly from the electric field reference data. This creates a significant difference signal and this is used to make an appropriate adjustment. Thus, as shown in a third histogram **1625,** the luminance of the image has been significantly increased and the saturation of the image has also been significantly increased. The hue remains unchanged such that the colour of the displayed region **1626** substantially retains its original colour.

Referring to the first histogram **1611,** the colour is substantially red and therefore appears red both in the original image and in the processed image. However, based upon the electric field difference values, the brightness of the image **1626** is increased and the saturation of the image is also increased. Adjustments to the saturation will make the red colour appear more red and less pink for example. An operative can clearly compare the modified image with the original image and from this make an assessment as to whether further intervention is required. Furthermore, additional prompts may be provided by the data processing system itself and a recommendation may also be made to the extent that the data should be sent to the remote data analysis system.

In alternative embodiments, different modifications to the image data could be made. For example, the hue of the image could be changed making the area of the visible skin condition appear in a very unsightly green colour, for example, when a malignant condition is suspected. Alternatively, the luminance value could be modified periodically to create a flashing or throbbing image; again, bringing the condition to the attention of an operative.

The system therefore presents a method of examining a visible skin condition in which a housing is located at a region of a visible skin condition. Image data is captured of the skin over the region and electric fields are generated that penetrate the skin over the region, such that an application of the apparatus produces both image related signals and electric field related signals for the same skin condition.

In an embodiment, a visual image is created that is derived from the image data and this image may be enhanced in response to the electric field data. In an embodiment, the test image data is compared against reference image data to identify an area of concern within the region, as shown in image **1622.** Furthermore, a keying operation may be performed to isolate the area of concern in the image data and colour properties of the area of concern may be changed in response to the electric field data.

### Figure 17

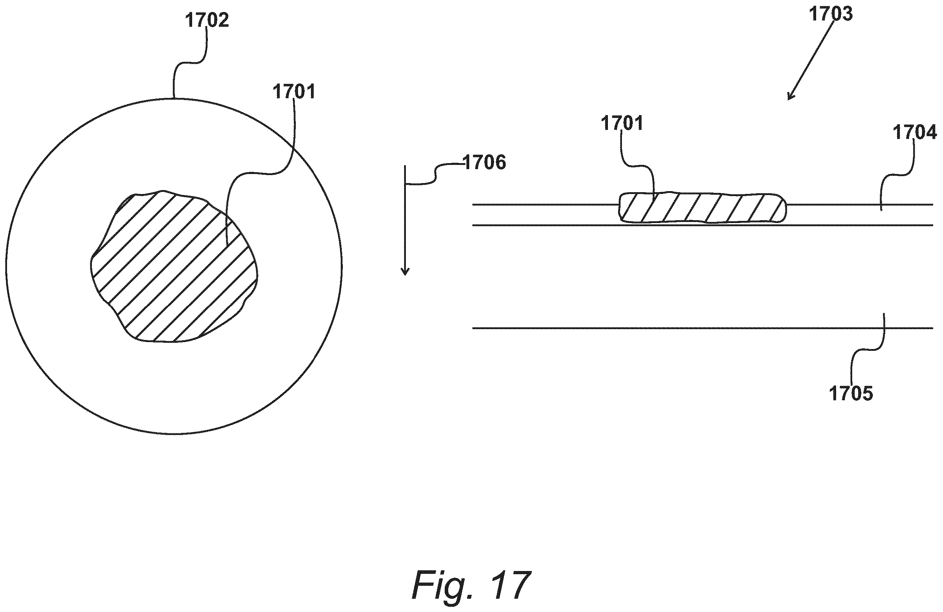

A visible skin condition **1701** as shown in *Figure 17*. The apparatus described with reference to *Figure 2* has been located over this visible skin condition and a captured image is bounded by circle a **1702.** For each deployment of the apparatus, the size of the visible image remains substantially constant and the number of pixels populated also remains constant. Consequently, the overall area of the image contained within the circle **1702** is known and may be represented in terms of the number of pixels present.

Following a keying operation, as described with reference to *Figure 16**,* it is possible for the area of the visible skin condition **1701** to be determined, as a subset of the available pixels, such that the area may be converted into conventional units or represented as a percentage of the overall area contained within circle **1702.** For the purposes of this illustration, it may be assumed that the visible skin condition **1701** is relatively large and, to a clinician, could be seen as problematic. The visible skin condition may be identified as a mole for example and the clinician would wish to know the extent to which the mole has penetrated the skin, representing the possibility of the undesirable material reaching a blood supply.

For the purposes of this illustration, a cross-section of the visible skin condition **1701** is shown at **1703.** The cross-section illustrates an epidermis **1704** and a dermis **1705.** In many situations, moles of this type would be considered problematic if they have, or if they possibly could, penetrate the dermis **1705.** However, in this example, the depth of the visible skin condition **1701,** as illustrated by a first arrow **1706,** is relatively shallow and has not entered the dermis **1705.** However, the captured image data does not provide this information and a clinician may be prompted to organise a biopsy. However, in the environment of *Figure 17*, this would be unnecessary and the clinician would be better informed if it were possible to obtain information indicating the depth of penetration without incurring a surgical procedure.

### Figure 18

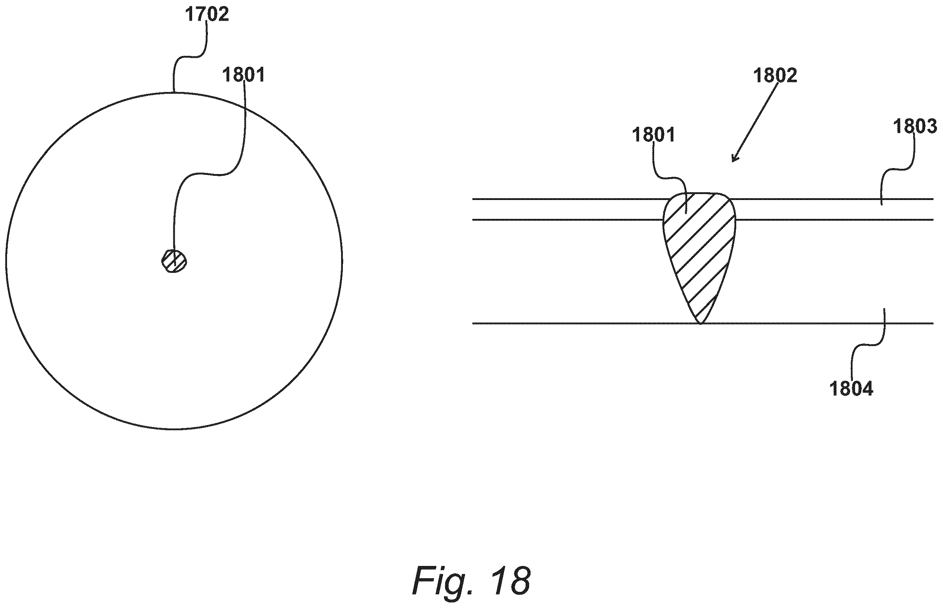

An image of a second visible skin condition **1801** is shown in *Figure 18**.* The overall area of the totality of the image is substantially the same as that described with reference to *Figure 17* and is again identified by circle **1702.** Again, the area of the second visible skin condition **1801,** which again may be identified as a mole, is much smaller than the area of the first visible skin condition **1701.** Thus, when presented only with the image data, a clinician may be tempted to conclude that the first visible skin condition **1701** appears more problematic than the second skin condition **1801.**

For the purposes of illustration, a cross-section of the second skin condition is shown at **1802,** with similar representations for an epidermis **1803** and a dermis **1804.** In this example, the second visible skin condition **1801** has penetrated through the epidermis **1803** and has also penetrated through most of the dermis **1804;** such that there is now a significant risk of the undesirable material reaching blood capillaries below the dermis **1804.** Thus, although from a visual inspection, the first visible skin condition **1701** appears more problematic than the second visible skin condition **1801,** in reality, due to the depth of penetration, the second visible skin condition **1801** is actually more problematic and would be assessed, following biopsy, as requiring surgical intervention.

### Figure 19

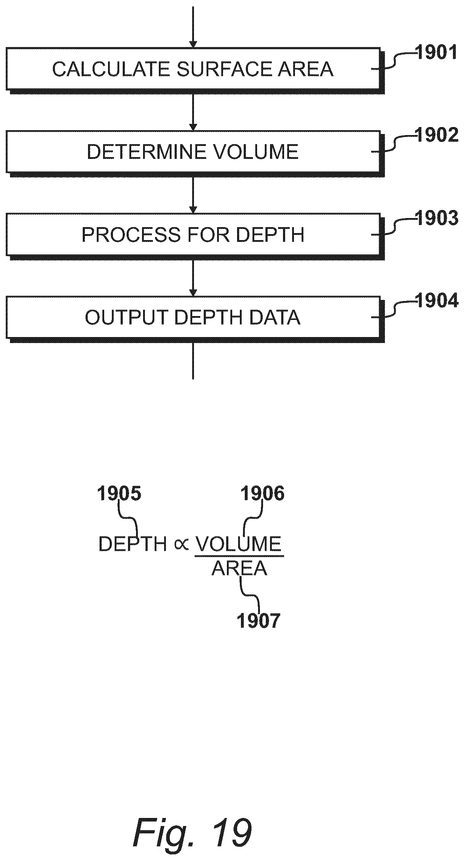

A further embodiment for step **1406** of processing the received data, as described with reference to *Figure 14*, is illustrated in *Figure 19*. In an embodiment, steps described with reference to *Figure 15* may also be incorporated.

At step **1901** the surface area of the visible skin condition is calculated. This makes use of the captured image data as described with reference to *Figure 17**.*

At step **1902,** the volume of the visible skin condition is determined from the electric field related data. Thereafter, at step **1903,** the calculated surface area is processed in combination with the determined indication of volume to assess the depth of skin penetration.

As illustrated in *Figure 3**,* the generated electric fields extend from the plane of the apparatus in three-dimensional space and thereby penetrate the skin. In an embodiment, this degree of penetration is such that it extends below the dermis **1705/1804.** As previously described, data is obtained by generating reference data which is then compared with the test data. The inventor has appreciated that the resulting electric field related signals represent, in the test data, values which vary with respect to the amount or volume of undesirable material present. Thus, the electric field data does not exclusively vary with respect to the viewable area of the skin condition but varies with respect to the totality of material present in the three-dimensional region comprising the epidermis **1803** and the dermis **1804.**

For the purposes of this illustration, it may be assumed that the volume of the undesirable material identified at **1701** is substantially similar to the volume of undesirable material identified at **1801.** In many practical implementations, it is unlikely that these assessments will be perfectly linear but in alternative embodiments, modifications may be made to the geometry of the apparatus to improve linearity, processing exercises may be performed to compensate for non-linear effects or reliance may be made upon a trained machine learning procedure. However, the realisation made by the inventor is to the effect that an indication of depth **1905** may be obtained by dividing the determined volume **1906** by the calculated surface area **1907.**

Following this procedure, the first visible skin condition **1703** producers a determined volume that is very similar to that of the second visible skin condition **1802.** However, the calculated area for the first visible skin condition **1701** is much larger than the calculated area for the second visible skin condition **1801,** therefore the assessment of depth **1905** will be significantly deeper for the second visible skin condition **1801.** Consequently, in a clinical environment, an operative would receive information to the effect that the second visible skin condition **1801** is actually likely to be more problematic than the first visible skin condition **1701.**

In an embodiment, deploying procedures substantially similar to those described with reference to *Figure 15* and *Figure 16*, modifications to the perceived colour of the visible skin conditions could be changed with reference to the depth assessment **1905.** Thus, for the purposes of illustration, it may be assumed that when viewing the captured image data, the first visible skin condition **1701** appears to have a colour that is substantially similar to the second visible skin condition **1801.** However, following the procedure described with reference to *Figure 19*, colour modifications may be made, as described with reference to *Figure 16**,* such that, when viewed, greater modifications are made to the colour of the second visible skin condition **1801** compared to the first visible skin condition **1701.**

### Figure 20

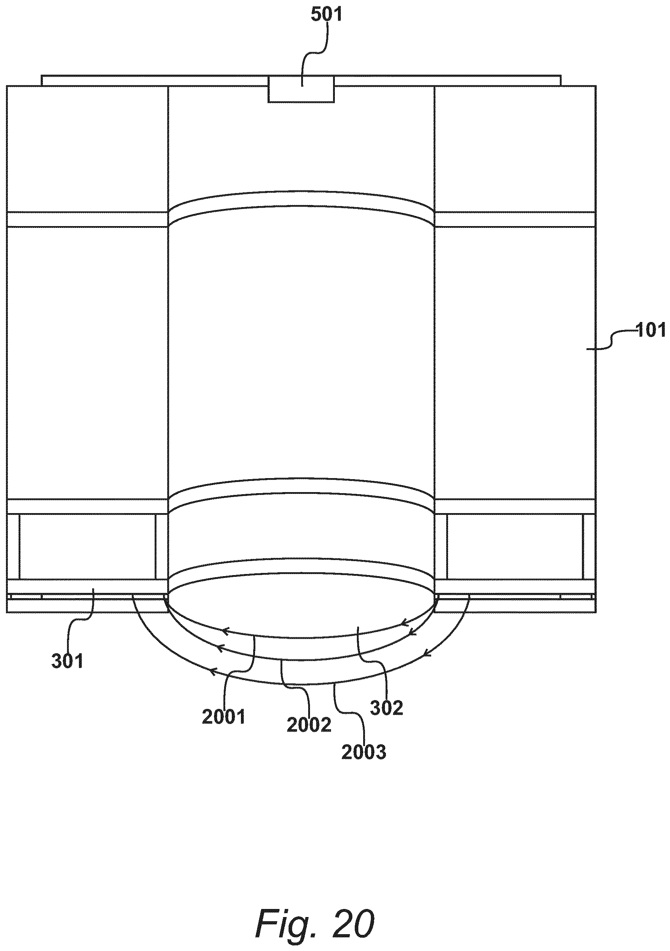

A cross-section of the apparatus of *Figure 3* is shown in *Figure 20**.* The housing **101** supports the first dielectric substrate **301** and the camera **501.** The visible skin condition is viewed through the hole **302** in the first dielectric substrate **301.** Scanning electrodes on the first dielectric substrate sequentially produce electric fields including a first electric field **2001.** This extends vertically from the housing **101** to penetrate the visible skin condition. Subsequently, a second electric field is generated that also penetrates the visible skin condition but with a greater degree of penetration. Similarly, a third electric field is generated with an even greater degree of skin penetration.

## Claims

1. An apparatus for examining visible skin conditions, comprising a housing locatable at a region of a skin condition, wherein said housing contains:
a camera (501) for capturing image data of the skin over said region; and
an electric field generating device (301, 311, 312) configured to generate electric fields (2001, 2002) that penetrate the skin over said region, such that an application of the apparatus produces both image related signals and electric field related signals for the same skin condition, **characterised in that**:
said electric field generating device comprises a first dielectric substrate (301) with a hole (302) having a first radius; and
said camera views the skin condition through said hole.

2. The apparatus of claim **1**, **characterised by** a processor configured to:
assess the depth of skin penetration of the visible skin condition; and
generate an output indicative of said assessing step.

3. The apparatus of claim **2, characterised in that** said processor is configured to:
calculate the surface area of the visible skin condition from the image data;
determine an indication of the volume of the visible skin condition from the electric field related signals; and
process said calculated surface area in combination with said determined indication of volume to assess the depth of skin penetration.

4. The apparatus of any of claims **1** to **3**, **characterised by** a plurality of circumferentially and substantially evenly displaced secondary electrodes (341, 342) on said first planar surface, wherein:
said secondary electrodes are positioned at a third radius; and
said third radius is larger than said second radius.

5. The apparatus of any of claims **1** to **4**, **characterised by** a signal processor for processing electric field signals produced by said electric field generating device, influenced by the electrical properties of the penetrated tissue, to produce electric field data.

6. The apparatus of claim **5**, **characterised by** a visual display device (1305) for showing the image data.

7. The apparatus of claim **6**, **characterised in that** a visual image (1305) derived from said image data is enhanced in response to said electric field data.

8. The apparatus of any of claims **1** to **7 characterised by** an analysing processor configured, following a machine learning exercise, to analyse said image data and said electric field data to produce an indication of a need for medical intervention with respect to the skin condition.

9. A method of examining a visible skin condition, comprising the steps of:
locating a housing (101) at a region of a visible skin condition (102);
capturing image data (1305) of the skin over said region; and
generating electric fields (2001) that penetrate the skin over said region, such that an application of the apparatus produces both image-related signals and electric field related signals for the same skin condition, **characterised by**:
assessing depth of skin penetration of the visible skin condition; and
generating an output indicative of said assessing step, by the steps of:
calculating (1901) the surface area of the visible skin condition from the image data produced by said capturing step;
determining (1902) an indication of the volume of the visible skin condition from the electric field related signals; and
processing (1903) said calculated surface area in combination with said determined indication of volume to assess the depth of skin penetration.

10. The method of claim **9**, **characterised in that**:
said generating step is performed by an electric field generating device that comprises a first dielectric substrate (301) having a hole (302) with a first radius;
said capturing step is performed by a camera (501); and
said camera views the visible skin condition through said hole.

11. The method of claim **2**, **characterised in that** image data is captured by the camera substantially at the same time as electric fields are generated by the electric field generating device.

12. The method of any of claims **9** to **11**, **characterised by** the step of locating the housing over an area of healthy skin to obtain reference signals for comparison against test signals.

13. The method of any of claims **9** to **12, characterised in that**:
the image data from said capturing step is displayed on a visual display device; and
said image data is enhanced in response to the electric field data.

14. The method of claim **13,** including the step of changing colour properties of the visible skin condition shown in the image data in response to an assessment of depth penetration.

15. The method of any of claims **9** to **14, characterised by** analysing the image data and the electric field data to produce an indication of a need for medical intervention with respect to the skin condition, following a machine learning exercise.
